# EUROPEAN PATENT SPECIFICATION

(11) **EP 1 381 352 B1**
(45) Date of publication and mention of the grant of the patent: **13.06.2007**
(21) Application number: 02715112.5
(22) Date of filing: 15.03.2002
(51) Int. Cl.: A61K 9/70, A61K 31/4468

(54) **TRANSDERMAL PATCH FOR ADMINISTERING FENTANYL**
TRANSDERMALES PFLASTER ZUR VERABREICHUNG VON FENTANYL
ADMINISTRATION TRANSDERMIQUE DE FENTANYLE ET DE SES ANALOGUES

(30) Priority: 16.03.2001 US 276837 P
(43) Date of publication of application: 21.01.2004
(60) Divisional application: 06018433.0
(73) Proprietor: Alza Corporation, Mountain View, CA 94039-7210 (US)
(72) Inventor: VENKATRAMAN, Subramanian, S., Palo Alto, CA 94303 (US); LI, Shaoling, Sunnyvale, CA 94086 (US); GALE, Robert, M., Los Altos, CA 94024 (US); STEPIC, Jane, San Carlos, CA 94070 (US); VAN OSDOL, William, W., Mountain View, CA 94040 (US)
(74) Representative: Williams, Paul Edwin
(86) International application number: PCT/US2002/007701
(87) International publication number: WO 2002/074286

(56) References cited:
- WO-A-99/32153
- US-A- 4 588 580
- US-A- 5 006 342
- US-A- 5 613 958
- US-A- 5 750 137
- US-B1- 6 203 817

## Description

### Field of the Invention

The present invention relates to a method and a patch for the transdermal administration of fentanyl and analogs thereof for analgetic purposes. In particular, the invention relates to a subsaturated patch for administering fentanyl and analogs thereof to a subject through skin over an extended period of time.

### Background of the Invention

Fentanyl and analogs thereof, such as alfentanil, carfentanil, lofentanil, remifentanil, sufentanil, trefentanil and the like, are powerful synthetic opioids which have demonstrated utility in both human and veterinary medicine. In human medicine, alfentanil, fentanyl, remifentanil and sufentanil have been granted regulatory approval for use as general anesthetics. A fentanyl containing lollipop for oral transmucosal administration and a fentanyl containing transdermal patch have also been approved as analgesics for use in the treatment of chronic pain.

The transdermal administration of these compounds for the treatment of both acute and chronic pain has been suggested and there are numerous patents describing various ways of transdermally administering fentanyl and analogs thereof. The following patents U.S. Patent Nos. 4,466,953; 4,470,962; 4,588,580; 4,626,539; 5,006,342; 5,186,939; 5,310,559; 5,474,783; 5,656,286; 5,762,952; 5,948,433; 5,985,317; 5,958,446; 5,993,849; 6,024,976; 6,063,399 and 6,139,866 are believed to be representative and are incorporated herein by reference. These patents disclose that fentanyl can be administered from a topically applied ointment or cream or from a transdermal patch.

A transdermal patch is typically a small adhesive bandage that contains the drug to be delivered and these bandages can take several forms. The simplest type is an adhesive monolith comprising a drug-containing reservoir disposed on a backing. The reservoir is typically formed from a pharmaceutically acceptable pressure sensitive adhesive but, in some cases, can be formed from a non-adhesive material, the skin-contacting surface of which is provided with a thin layer of a suitable adhesive. The rate at which the drug is administered to the patient from these patches can vary due to normal person-to-person and skin site-to-skin site variations in the permeability of skin to the drug.

More complex patches are multilaminate or liquid reservoir types of patches in which a drug release-rate controlling membrane is disposed between the drug reservoir and the skin-contacting adhesive. This membrane, by decreasing the in vitro release rate of drug from the patch, serves to reduce the effects of variations in skin permeability. This type of patch is generally preferred when a highly potent drug is being administered but has the disadvantage of usually having to cover a larger area of skin than a monolithic patch to achieve the same drug administration rate.

The drug reservoirs of transdermal patches can have the drug either completely dissolved in the reservoir (subsaturated patches, see e.g., U.S. Pat. Nos., 4,704,282; 4,725,439; 4,867,982; 4,908,027; 5,004,610; 5,152,997; 5,164,190; 5,342,623; 5,344,656; 5,364,630; 5,462,745; 5,633,008 and 6,165,497) or can contain an excess of undissolved drug over the saturation concentration (depot patches). Because transdermal patches deliver drug by diffusion through the skin, the delivery rate of the drug from the patch is governed by Fick's law and is proportional to the level of saturation of the drug in the reservoir.

In a depot patch, the excess drug allows the reservoir to remain saturated with the drug after the patch is applied and it can deliver the drug at the greatest rate for as long as the excess is present. A subsaturated patch, however, will typically exhibit a continuous decrease in the degree of saturation of the drug in the reservoir and the administration rate of the drug will tend to decrease continuously during use. Thus, depot patches would be preferred where a relatively constant drug administration rate is desired, but the presence of undissolved drug or other constituents in a patch can cause stability and other problems during storage and use.

Fentanyl and analogs thereof are potent opioids having relatively narrow therapeutic indices. Being potent means that relatively low concentrations of the drug in the blood are sufficient to produce the desired effect. Having a narrow therapeutic index means that the therapeutic effect is obtained only over a narrow range of concentrations; concentrations below the range being ineffective and concentrations above the range being associated with serious, and in the case of opioids, potential lethal side effects. This combination of characteristics, coupled with the patient-to-patient variations in response to opioid analgesics, dictates extreme caution in the administration of opioid drugs.

Because of the wide variations in individual pharmacokinetic (e.g., drug clearance rates) and pharmacodynamic response to opioids (e.g., the subjective nature of pain and the danger associated with overdose), patients typically need to be titrated upwards to determine the appropriate dose. This means that a patient is initiated at a dose that is expected to be safe and the dose is gradually increased until adequate analgesia is obtained. Because with time, both tolerance to opioids and increased severity of pain may occur, doses may be subsequently increased and/or supplemented with doses of other analgesics for the management of pain. In addition, some patients will require the rescue use of another opioid for the treatment of episodes of breakthrough pain along with their baseline treatment with transdermal opioids.
US 5,588,580 discloses transdermal patches for the administration of fentanyl, or its derivatives, in which a drug reservoir composition comprises an aqueous or nonaqueous gelling-system comprising ethanol, water and a gelling agent, or polyisobutylene plasticised with mineral oil respectively.
WO 02/26217 discloses transdermal patches comprising a reservoir and a backing layer for fentanyl delivery over a period of 4-14 days.

Although the analgetic transdermal administration of fentanyl and analogs thereof has been widely suggested In the prior art, using transdermal patches of the various types described above, only one such product has actually received regulatory approval In the United States. This product, DURAGESIC^{®}, is a patch that administers fentanyl for 3 days and is indicated for the treatment of chronic pain, as opposed to post-operative or other acute pain. A copy of the labeling describing this patch and its use is incorporated by reference herein (Physicians Desk Reference, 56th Edition, 2002, pages 1786-1789). The DURAGESIC^{®} fentanyl patch is intended to be sequentially removed and replaced with a fresh patch at the end of each 3 day period to provide relief from chronic pain and it is contemplated that doses may be increased over time and that concurrent use of other analgesics may occur to deal with breakthrough pain.

Because of fentanyl's high potency and narrow therapeutic index, DURAGESIC^{®} fentanyl system was designed as a rate controlled, liquid reservoir, depot patch of the type described in Examples 1-4 of US patent 4,588,580.

We have now discovered that fentanyl and analogs thereof can be safely and analgetically effectively delivered over periods of at least 3 days from non-rate controlled, monolithic, subsaturated patches having the characteristics hereinafter described. As a result, fabrication of the patch is simplified, stability of the patch improved and a more comfortable, patient friendly patch provided.

We have also provided a non-rate controlled, monolithic subsaturated patch that is bioequivalent or pharmacologically equivalent to the liquid reservoir, rate-controlled, depot DURAGESIC^{®} transdermal fentanyl patch.

### Definitions

In describing the present invention, the following terms will be employed, and are intended to be defined as indicated below. As used in this specification and the appended claims, the singular forms "a," "an" and "the" include plural references unless the content clearly dictates otherwise.

As used herein, the term "an analog of fentanyl" (hereafter referred to as "analog") refers to extremely potent and effective analgesics such alfentanil, carfentanil, lofentanil, remifentanil, sufentanil, trefentanil, and the like.

As used herein, the term "drug" refers to fentanyl and analogs thereof.

As used herein, the term "subsaturated patch" refers to patch wherein the concentration of the drug is below its solubility limit. The drug reservoir comprises a single phase polymeric composition, free of undissolved components, wherein the drug and all other components are present at concentrations no greater than, and preferably less than, their saturation concentrations in the reservoir.

As used herein, the term "single phase polymeric composition" refers to a composition in which the drug and all other components are solubilized in a polymer and are present at concentrations no greater than, and preferably less than, their saturation concentrations in the reservoir such that there are no undissolved components present in the composition over a substantial portion of the administration period; wherein all the components in combination with the polymer form a single phase.

As used herein, the term "component" refers to an element within the drug reservoir, including, but not limited to, a drug as defined above, additives, permeation enhancers, stabilizers, dyes, diluents, plasticizer, tackifying agent, pigments, carriers, inert fillers, antioxidants, excipients, gelling agents, anti-irritants, vasoconstrictors and the like.

As used herein, a "rate controlling membrane" refers to a drug release-rate controlling membrane as discussed above.

A "DURAGESIC^{®} fentanyl patch" refers to a fentanyl patch as discussed above (see also Physicians Desk Reference, 56th Edition, 2002, pages 1786-1789).

As used herein, the term "Cₘₐₓ" refers to the peak blood or plasma concentration of the drug, i.e., fentanyl or the analog thereof.

As used herein, the term "standardized Cₘₐₓ (ng/ml-cm²)" refers to the Cₘₐₓ (ng/ml) per unit area (cm²) of the active drug delivery area of the system, e.g., the area of the drug reservoir.

As used herein, the term "normalized Cₘₐₓ (ng/ml-(mg/h))" refers to the Cₘₐₓ (ng/ml) divided by the rate of the drug administered (mg/h).

As used herein, the term "steady state drug flux" refers to the drug flux (in vitro and in vivo) in the range of 1 to 20 µg/h-cm² over a substantial portion of the administration period.

As used herein, the term "bioavailability", refers to the rate and extent to which the active ingredient or active moiety is absorbed from a drug product and becomes available at the site of action. The rate and extent are established by the pharmacokinetic-parameters, such as, the area under the blood or plasma drug concentration-time curve (AUC) and the peak blood or plasma concentration (Cₘₐₓ) of the drug.

Two different products are considered to be "bioequivalent" if they produce substantially the same pharmacokinetic effects when studied under similar experimental conditions. Bioequivalence may be demonstrated through several in vivo and in vitro methods. These methods, in descending order of preference, include pharmacokinetic, pharmacodynamic, clinical and in vitro studies. In particular, bioequivalence is demonstrated using pharmacokinetic measures such as the area under the blood or plasma drug concentration-time curve (AUC) and the peak blood or plasma concentration (Cₘₐₓ) of the drug, using statistical criteria as described in greater detail hereinafter.

Two different products are considered to be "pharmacologically equivalent" if they produce substantially the same therapeutic effects when studied under similar experimental conditions, as demonstrated through several in vivo and in vitro methods as described in greater detail hereinafter. Therapeutic effects depend on various factors, such as, potency of the drug, the solubility and diffusivity of the drug In the skin, thickness of the skin, concentration of the drug within the skin application site, concentration of the drug in the drug reservoir, and the like, as described In greater detail hereinafter. In general, pharmacological equivalence Is demonstrated using measures such as the peak blood or plasma concentration of the drug normalized for the rate of drug administered (i.e. normalized Cₘₐₓ as defined above) and the peak blood or plasma concentration of the drug standardized per unit area of the active drug delivery area of the system (i.e. standardized Cₘₐₓ as defined above).

When comparing two different products whose drug administration rate is proportional to the size of the patch, bioequivalence or pharmacological equivalence may be established either by normalizing the peak blood or plasma concentration of the drug (Cₘₐₓ) for the rate of drug administered (normalized Cₘₐₓ), or by standardizing the peak blood or plasma concentration of the drug (Cₘₐₓ) per unit area of the active drug delivery area of the system (standardized Cₘₐₓ). However, when comparing two different products having different drug administration rate per unit area, it is necessary to normalize the peak blood or plasma concentration of the drug (Cₘₐₓ) on the basis of the rate of drug administered to establish bioequivalence or pharmacological equivalence.

### Summary of the Invention

The present invention provides a transdermal patch for administering fentanyl, alfentanil, carfentanil, lofentanil, remifentanil, sufentanil or trefentanil through the skin comprising:
(a) a backing layer;
(b) a reservoir disposed on the backing layer, at least the skin contacting surface of said reservoir being adhesive; said reservoir comprising a single phase polymeric composition free of undissolved components containing an amount of a drug selected from the group consisting of fentanyl, alfentanil, carfentanil, lofentanil, remifentanil, sufentanil and trefentanil sufficient to induce and maintain analgesia in a human for at least three days;
characterised in that the reservoir is formed from a polyacrylate adhesive and has a thickness of 0.0125 mm (0.5 mil) to 0.1 mm (4 mil).

The present invention provides a patch for transdermal delivery of fentanyl and analogs thereof for analgetic purposes, to a subject through skin over an extended period of time. In particular, the present invention provides a non-rate controlled, monolithic, subsaturated patch for transdermal delivery of fentanyl and analogs thereof at an administration rate sufficient to induce and maintain analgesia for at least three days. In preferred embodiments, the drug is fentanyl, preferably, base form of fentanyl. In additionally preferred embodiments, the drug is sufentanil, preferably the base form of sufentanil.

In another aspect, the present invention provides a non-rate controlled, monolithic subsaturated patch that is bioequivalent to the liquid reservoir, rate-controlled, depot DURAGESIC^{®} fentanyl patch. In an alternative aspect, the present invention provides a non-rate controlled, monolithic subsaturated patch that is pharmacologically equivalent to the liquid reservoir, rate-controlled, depot DURAGESIC^{®} fentanyl patch.

In an additional aspect, the invention pertains to a transdermal patch for administering drug through the skin comprising: (a) a backing layer; and (b) a reservoir disposed on the backing layer, at least the skin contacting surface of the reservoir being adhesive; wherein the reservoir comprises a single phase polymeric composition free of undissolved components containing an amount of the drug sufficient to induce and maintain analgesia for at least three days.

These and other embodiments of the present invention will readily occur to those of ordinary skill in the art in view of the disclosure herein.

### Brief Description of the Figures

Figure 1 illustrates a cross-section through a schematic, perspective view of one embodiment of transdermal therapeutic system according to this invention.

Figure 2 illustrates a cross-section view through another embodiment of this invention.

Figure 3 illustrates the in vitro transdermal flux of various fentanyl patches.

Figure 4 illustrates the in vitro transdermal flux of various fentanyl and sufentanil patches.

Figure 5 illustrates the in vitro transdermal flux of various fentanyl and sufentanil patches.

Figure 6 illustrates the in vitro transdermal flux of various fentanyl and sufentanil patches as a function of drug loading.

Figure 7 illustrates serum fentanyl concentrations following transdermal application of various fentanyl patches for 72 hours, over a 96 hour period post application.

Figure 8 illustrates serum fentanyl concentrations following transdermal application of various fentanyl patches for 72 hours, over a 120 hour period post application.

### Detailed Description of the Invention

The practice of the present invention will employ, unless otherwise indicated, conventional methods used by those in pharmaceutical product development within those of skill of the art. Such techniques are explained fully in the literature. See, e.g., Patini, G.A. and Chein, Y.W., Swarbrick, J. and Boylan, J.C., eds, Encyclopedia of Pharmaceutical Technology, New York: Marcel Dekker, Inc., 1999 and Gale, R., Hunt, J. and Prevo, M., Mathiowitz, E., ed, Encyclopedia of Controlled Drug Delivery Patches, Passive, New York: J Wiley & Sons, Inc, 1999.

### Modes of Carrying Out the invention

The present Invention provides a patch for transdermal delivery of fentanyl and analogs thereof for analgetic purposes, to a subject through skin over an extended period of time. In particular, the present invention provides a non-rate controlled, monolithic, subsaturated patch for transdermal delivery of fentanyl and analogs thereof at a rate and In an amount sufficient to induce and maintain analgesia over a period of at least three days, and up to 7 days to a patent in need thereof.

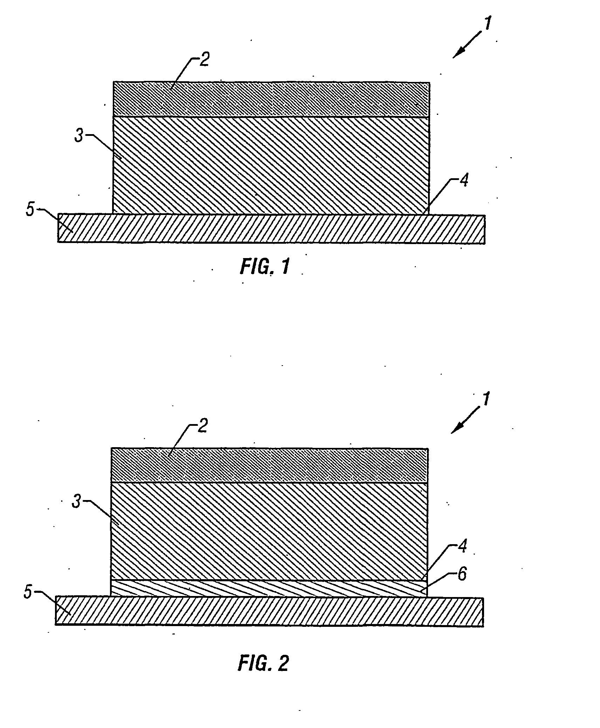

Referring now to Figures 1 and 2, a preferred embodiment of the transdermal monolithic patch 1 according to this invention comprises a backing layer 2, a drug reservoir 3 disposed on the backing layer 2, wherein at least the skin contacting surface 4 of the reservoir 3 is adhesive, and a peelable protective layer 5. The reservoir 3 comprises a single phase polymeric composition in which the drug and all other components are present at concentrations no greater than, and preferably less than, their saturation concentrations in the reservoir 3. This produces a composition in which no undissolved components are present. In preferred embodiments, the reservoir 3 is formed from a pharmaceutically acceptable adhesive.

Referring now to Figure 2, the reservoir 3 is formed from a material that does not have adequate adhesive properties, In this embodiment of a monolithic patch 1, the skin contacting surface of the reservoir 4 may be formulated with a thin adhesive coating 6. The reservoir 3 is a single phase polymeric composition as described earlier.

The backing layer 2 may be a breathable or occlusive material comprising fabric, polyvinyl acetate, polyvinylidene chloride, polyethylene, polyurethane, polyester, ethylene vinyl acetate (EVA), polyethylene terephthalate, polybutylene terephthalate, coated paper products, aluminum sheet and the like, and a combination thereof. In preferred embodiments, the backing layer comprises low density polyethylene (LDPE) materials, medium density polyethylene (MDPE) materials or high density polyethylene (HDPE) materials, e.g., SARANEX (Dow Chemical, Midland, MI). The backing layer may be a monolithic or a multilaminate layer. In preferred embodiments, the backing layer is a multilaminate layer comprising nonlinear LDPE layer/linear LDPE layer/nonlinear LDPE layer. The backing layer has a thickness of 0.012 mm (0.5 mil) to 0.125 mm (5 mil); preferably 0.025 mm (1 mil) to 0.1 mm (4 mil); more preferably 0.0625 mm (1.5 mil) to 0.0875 mm (3.5 mil).

The drug reservoir 3 is disposed on the backing layer, wherein at least the skin contacting surface of the reservoir is adhesive.

The reservoir is formed from a pharmaceutically acceptable polyacrylate adhesive, as described in greater detail below.

The acrylic polymers are comprised of a copolymer or terpolymer comprising at least two or more exemplary components selected from the group comprising acrylic acids, alkyl acrylates, methacrylates, copolymerizable secondary monomers or monomers with functional groups. Examples of monomers include, but are not limited to, acrylic acid, methacrylic acid, methoxyethyl acrylate, ethyl acrylate, butyl acrylate, butyl methacrylate, hexyl acrylate, hexyl methacrylate, 2-ethylbutyl acrylate, 2-ethylbutyl methacrylate, isooctyl acrylate, isooctyl methacrylate, 2-ethylhexyl acrylate, 2-ethylhexyl methacrylate, decyl acrylate, decyl methacrylate, dodecyl acrylate, dodecyl methacrylate, tridecyl acrylate, tridecyl methacrylate, hydroxyethyl acrylate, hydroxypropyl acrylate, acrylamide, dimethylacrylamide, acrylonitrile, dimethylaminoethyl acrylate, dimethylaminoethyl methacrylate, tert-butylaminoethyl acrylate, tert-butylaminoethyl methacrylate, methoxyethyl acrylate, methoxyethyl methacrylate, and the like. Additional examples of appropriate acrylic adhesives suitable in the practice of the invention are described in Satas, "Acrylic Adhesives," Handbook of pressure-Sensitive Adhesive Technology, 2nd ed., pp. 396-456 (D. Satas, ed.), Van Nostrand Reinhold, New York (1989). The acrylic adhesives are commercially available (National Starch and Chemical Corporation, Bridgewater, NJ; Solutia, MA). Further examples of polyacrylate-based adhesives are as follows, identified as product numbers, manufactured by National Starch (Product Bulletin, 2000): 87-4098, 87-2287, 87-4287, 87-5216, 87-2051, 87-2052, 87-2054, 87-2196, 87-9259, 87-9261, 87-2979, 87-2510, 87-2353, 87-2100, 87-2852, 87-2074, 87-2258, 87-9085, 87-9301 and 87-5298.

The acrylic polymers comprise cross-linked and non-cross-linked polymers. The polymers are cross-linked by known methods to provide the desired polymers. In preferred embodiments, the adhesive is a polyacrylate adhesive having a glass transition temperature (T_{g}) less than -10°C, more preferably having a T_{g} of -20°C to -30 °C. The molecular weight of the polyacrylate adhesive, expressed as weight average (MW), generally ranges from 25,000 to 10,000,000, preferably from 50.000 to 3,000,000 and more preferably from 100,000 to 1,000,000 prior to any cross-linking reactions. Upon cross-linking the MW approaches infinity, as known to those involved in the art of polymer chemistry.

As discussed above, the reservoir 3 comprises a single phase polymeric composition, free of undissolved components, containing an amount of the drug sufficient to induce and maintain analgesia in a human for at least three days. The drug is selected from a group consisting of fentanyl and analogs thereof, such as, alfentanil, carfentanil, lofentanil, remifentanil, sufentanil, trefentanil, and the like. In preferred embodiments, the drug reservoir comprises 0.05 to 1.75 mg/cm² of the drug; preferably 0.07 to 1.50 mg/cm² of the drug; preferably 0.08 to 1.25 mg/cm² of the drug; more preferably 0.09 to 1.0 mg/cm² of the drug; more preferably, 0.1 to 0.75 mg/cm² of the drug; and even more preferably 0.12 to 0.5 mg/cm² of the drug. The drug should be soluble in the polymer forming reservoir 3 in a form that is as discussed below. In preferred embodiments, the drug is in the base form and the preferred drugs are fentanyl or sufentanil. In particularly preferred embodiments, the drug reservoir comprises 0.05 to 1.75 mg/cm² of fentanyl; preferably 0.07 to 1.50 mg/cm² of fentanyl; preferably 0.08 to 1.25 mg/cm² of fentanyl; more preferably about 0.09 to about 1.0 mg/cm² of fentanyl; more preferably about 0.1 to 0.75 mg/cm² of fentanyl; and even more preferably 0.12 to 0.5 mg/cm² of fentanyl; wherein fentanyl is in a base form and is completely dissolved. In additionally preferred embodiments, the drug reservoir comprises 0.05 to 1.75 mg/cm² of sufentanil; preferably 0.07 to 1.50 mg/cm² of sufentanil; preferably 0.08 to 1.25 mg/cm² of sufentanil; more preferably 0.09 to 1.0 mg/cm² of sufentanil; more preferably 0.1 to 0.75 mg/cm² of sufentanil; more preferably 0.12 to 0.5 mg/cm² of sufentanil; and even more preferably 0.25 to 0.4 mg/cm² of sufentanil; wherein sufentanil is in a base form and is completely dissolved.

The material forming the reservoir 3 has a solubility for the drug of 1 wt% to 25 wt% of the total polymer composition; preferably 2 wt% to 15 wt%; more preferably 4 wt% to 12 wt% of the total polymer composition; and even more preferably 6 wt% to 10 wt% of the total polymer composition. The reservoir 3, with or without the adhesive coating 6, has a thickness of 0.0125 mm (0.5 mil) to 0.1 mm (4 mil); preferably 0.025 mm (1 mil) to 0.0875 mm (3.5 mil); more preferably 0.0375 mm (1.5 mil) to 0.075 (3 mil); and even more preferably 0.04 mm (1.6 mil) to 0.05 mm (2 mil). In preferred embodiments, the drug is fentanyl, preferably in the base form, wherein the material forming the reservoir 3 has a solubility for fentanyl of 1 wt% to 25 wt% of the total polymer composition; preferably 3 wt% to 15 wt%; more preferably 5 wt% to 12 wt%; and even more preferably 7 wt% to 10 wt% of the total polymer composition. The reservoir 3, with or without the adhesive coating 6, has a thickness of 0.0125 mm (0.5 mil) to 0.1 mm (4 mil); preferably 0.025 mm (1 mil) to 0.075 mm (3 mil); more preferably 0.0375 mm (1.5 mil) to 0.0625 (2.5 mil); and even more preferably 0.04 mm (1.6 mil) to 0.05 mm (2 mil). In additionally preferred embodiments, the drug is sufentanil, preferably in the base form, wherein the material forming the reservoir 3 has a solubility for sufentanil of 1 wt% to 25 wt% of the total polymer composition; preferably 3 wt% to 15 wt%; more preferably 5 wt% to 12 wt%; and even more preferably 7 wt% to 10 wt% of the total polymer composition. The reservoir 3, with or without the adhesive coating 6, has a thickness of 0.0125 mm (0.5 mil) to 0.1 mm (4 mil); preferably 0.025 mm (1 mil) to 0.075 mm (3 mil); more preferably 0.0375 mm (1.5 mil) to 0.0625 (2.5 mil); and even more preferably 0.04mm (1.6 mil) to 0.5 mm (2 mil).

In additional embodiments, the reservoir 3 may optionally contain additional components such as, additives, permeation enhancers, stabilizers, dyes, diluents, plasticizer, tackifying agent, pigments, carriers, inert fillers, antioxidants, excipients, gelling agents, anti-irritants, vasoconstrictors and other materials as are generally known to the transdermal art, provided that such materials are present below saturation concentration in the reservoir.

Examples of permeation enhancers include, but are not limited to, fatty acid esters of glycerin, such as capric, caprylic, dodecyl, oleic acids; fatty acid esters of isosorbide, sucrose, polyethylene glycol; caproyl lactylic acid; laureth-2; laureth-2 acetate; laureth-2 benzoate; laureth-3 carboxylic acid; laureth-4; laureth-5 carboxylic acid; oleth-2; glyceryl pyroglutamate oleate; glyceryl oleate; N-lauroyl sarcosine; N-myristoyl sarcosine; N-octyl-2-pyrrolidone; lauraminopropionic acid; polypropylene glycol-4-laureth-2; polypropylene glycol-4-laureth-5dimethyl lauramide; lauramide diethanolamine (DEA). Preferred enhancers include, but are not limited to, lauryl pyroglutamate (LP), glyceryl monolaurate (GML), glyceryl monocaprylate, glyceryl monocaprate, glyceryl monooleate (GMO) and sorbitan monolaurate. Additional examples of suitable permeation enhancers are described, for example, in U.S. Patent Nos.: 5,785,991; 5,843,468; 5,882,676; and 6,004,578.

In certain embodiments, the reservoir comprises diluent materials capable of reducing quick tack, increasing viscosity, and/or toughening the matrix structure, such as polybutylmethacrylate (ELVACITE, manufactured by ICI Acrylics, e.g., ELVACITE 1010, ELVACITE 1020, ELVACITE 20), high molecular weight acrylates, i.e., acrylates having an average molecular weight of at least 500,000, and the like.

In certain embodiments, a plasticizer or tackifying agent is incorporated in the adhesive composition to improve the adhesive characteristics. Examples of suitable tackifying agents include, but are not limited to, aliphatic hydrocarbons; aromatic hydrocarbons; hydrogenated esters; polyterpenes; hydrogenated wood resins; tackifying resins such as ESCOREZ, aliphatic hydrocarbon resins made from cationic polymerization of petrochemical feedstocks or the thermal polymerization and subsequent hydrogenation of petrochemical feedstocks, rosin ester tackifiers, and the like; mineral oil and combinations thereof.

The tackifying agent employed should be compatible with the blend of polymers. For example, the styrenic block copolymers can be formulated with rubber compatible tackifying resins, end-block compatible resins such polymethyl styrene, or plasticizers such as mineral oil. Generally the polymer is 5-50% of the total adhesive composition, the tackifier is 30-85% of the total adhesive composition, and the mineral oil is 2-40% of total adhesive composition.

The patch 1 further comprises a peelable protective layer 5. The protective layer 5 is made of a polymeric material that may be optionally metallized. Examples of the polymeric materials include polyurethane, polyvinyl acetate, polyvinylidene chloride, polypropylene, polycarbonate, polystyrene, polyethylene, polyethylene terephthalate, polybutylene terephthalate, paper, and the like, and a combination thereof. In preferred embodiments, the protective layer comprises a siliconized polyester sheet.

A wide variety of materials which can be used for fabricating the various layers of the transdermal delivery patches according to this invention have been described above. This invention therefore contemplates the use of materials other than those specifically disclosed herein, including those which may hereafter become known to the art to be capable of performing the necessary functions.

### Administration of the Drug

On application to the skin, the drug in the drug reservoir 3 of the transdermal patch 1 diffuses into the skin where it is absorbed into the bloodstream to produce a systemic analgetic effect. The onset of analgesia depends on various factors, such as, potency of the drug, the solubility and diffusivity of the drug in the skin, thickness of the skin, concentration of the drug within the skin application site, concentration of the drug in the drug reservoir, and the like (see e.g., U.S. Patent No. 4,588,580 for a discussion of relative permeabilities and potencies of fentanyl and analogs thereof). It is preferable that a patient experience an adequate effect within six hours of initial, application. However, this is significant only on the initial application. On repeated sequential application, the residual drug in the application site of the patch is absorbed by the body at approximately the same rate as the drug from the new patch is absorbed into the new application area. Thus the patient should not experience any Interruption of analgesia.

The concentration of the drug within the skin application sites are also significant in establishing an upper limit on the size of the transdermal therapeutic patch and, conversely, the lower limit on the usable administration rate. In general, when patch according to this invention is employed, the total amount of drug within the skin application site of the patch ranges from 0.05 to = 200 µg/cm². When such a patch is removed, the analgesic effect continues until the amount of residual drug in the skin is reduced sufficiently below the minimum effective plasma concentration of the drug. For example, after removal of a fentanyl patch, the serum concentrations of fentanyl decline gradually and reach a 50% reduction in serum levels in approximately 17 hours (see e.g., the labeling insert for the DURAGESIC® patch). These amounts will vary for other drugs, depending on the solubility of the drug and the size of the patch. For example, the solubility of sufentanil in the epidermis is up to 25% to 50% of fentanyl. In view of the high potency of fentanyl and analogs thereof, preferably the amount of drug solubilized In the skin is maintained at an appropriate level to permit prompt termination of therapy.

When continuous analgesia is desired the depleted patch would be removed and a fresh patch is applied to a new location. For example, the patch would be sequentially removed and replaced with a fresh patch at the end of the administration period to provide relief from chronic pain. Since absorption of the drug from the fresh patch into the new application area usually occurs at substantially the same rate as absorption by the body of the residual drug within the previous application site of the patch, blood levels will remain substantially constant. Additionally, it is contemplated that doses may be increased over time and that concurrent use of other analgesics may occur to deal with breakthrough pain.

In preferred embodiments, the invention provides for a transdermal patch exhibiting a normalized Cₘₐₓ ranging from 3.3 to 82.5 ng/ml-(mg/h), preferably 6.6 to 50 ng/ml-(mg/h), more preferably 13 to 40 ng/ml-(mg/h), and even more preferably from 20 to 35 ng/ml-(mg/h); and a standardized Cₘₐₓ ranging from 0.001 to 0.2 ng/ml-cm², preferably 0.005 to 0.15 ng/ml-cm², more preferably 0.008 to 0.1 ng/ml-cm², and even more preferably from 0.01 to 0.08 ng/ml-cm². The transdermal patch is 0.5 to 150 cm²; preferably 2 to 100 cm²; more preferably 4 to 50 cm², and even more preferably 10 to 20 cm². On administration over skin the transdermal patch exhibits a steady state drug flux of 0.1 to 20 µg/cm²/hr; preferably 0.75 to 10 µg/cm²/hr; preferably 1 to 8 µg/cm²/hr; more preferably about 1.5 to about 5 µg/cm²/hr; more preferably about 2 to about 3 µg/cm²/hr, and even more preferably 1 to 2.5 µg/cm²/hr. Steady-state administration rates obtainable according to this invention range from 0.1 to 500 µg/h; preferably 1 to 300 µg/h; more preferably 2 to 250 µg/h; and even more preferably 5 to 200 µg/h.

In additionally preferred embodiments, the invention provides for a transdermal fentanyl patch exhibiting a normalized Cₘₐₓ ranging from 3.3 to 82.5 ng/ml-(mg/h), preferably 10 to 62 ng/ml-(mg/h), more preferably from 16 to 41 ng/ml-(mg/h), and even more preferably from 20 to 35 ng/ml-(mg/h); and a standardized Cₘₐₓ ranging from 0.01 to 0.2 ng/ml-cm², preferably 0.02 to 0.15 ng/ml-cm², more preferably from 0.03 to 0.1 ng/ml-cm², and even more preferably from 0.04 to . 0.08 ng/ml-cm². The transdermal fentanyl patch is 1 to 150 cm²; preferably 2 to 125 cm²; more preferably 4 to 100 cm²; more preferably 5 to 75 cm², and even more preferably 5 to 50 cm². On administration over skin, the transdermal fentanyl patch exhibits a steady state drug flux of 1 to 10 µg/cm²/hr; preferably 1.5 to 8 µg/cm²/hr; more preferably 2 to 5 µg/cm²/hr, and even more preferably 2 to 3 µg/cm²/hr. Steady-state administration rates obtainable for a fentanyl patch according to this invention range from 1 to 300 µg/h; preferably 2 to 250 µg/h; and more preferably 5 to 200 µg/h.

In additionally preferred embodiments, the invention provides for a transdermal sufentanil patch exhibiting a normalized Cₘₐₓ ranging from 0.04 to 10 ng/ml-(mg/h), preferably 1 to 8 ng/ml-(mg/h), and more preferably from 2 to 5.5 ng/ml-(mg/h), and even more preferably 2.5 to 5 ng/ml-(mg/h); and a standardized Cₘₐₓ ranging from 0.001 to 0.05 ng/ml-cm², preferably 0.005 to 0.04 ng/ml-cm², more preferably from 0.0075 to 0.025 ng/ml-cm², and more preferably from 0.01 to 0.02 ng/ml-cm². the transdermal sufentanil patch is 0.5 to 40 cm²; preferably 1 to 35 cm²; and more preferably about 2 to about 30 cm². On administration over skin, the transdermal sufentanil patch exhibits a steady state drug flux of 0.1 to 10 µg/cm²/hr; preferably 0.5 to 8 µg/cm²/hr; more preferably 0.75 to 6 µg/cm²/hr; more preferably 1 to 5 µg/cm²/hr; and even more preferably 1 to 2.5 µg/cm²/hr. Steady-state administration rates obtainable for a sufentanil patch according to this invention range from 0.1 to 200 µg/h; preferably 0.25 to 150 µg/h; more preferably 0.5 to 100 µg/h; more preferably 0.75 to 50 µg/h; and even more preferably 1 to 40 µg/h.

Administration is maintained for at least three days, and up to 7 days, with 3-4 day regimen being considered preferable. In preferred embodiments, at least 3%, but not more than 40%, of the total amount of the drug In the patch is administered during approximately the first 24 hours of use; at least 6%, but not more than 50%, of the total amount of the drug is administered during approximately the first 48 hours of use; and at least 10%, but not more than 75%, of the total amount of the drug is administered during the administration period. In preferred embodiments, the patch is a fentanyl patch wherein at least 5%, but not more than 40%, of the total amount of the drug in the patch is administered during approximately the first 24 hours of use; at least 15%, but not more than 50%, of the total amount of the drug is administered during approximately the first 48 hours of use; and at least 25%, but not more than 75%, of the total amount of the drug is administered during the administration period. In alternative embodiments, the patch is a sufentanil patch wherein at least 3%, but not more than 40%, of the total amount of the drug in the patch is administered during approximately the first 24 hours of use; at least 6%, but not more than 50%, of the total amount of the drug is administered during approximately 48 hours of use; and at least 10%, but not more than 75%, of the total amount of the drug is administered during the administration period.

A preferred embodiment of this invention is a patch that is bioequivalent to the DURAGESIC^{®} fentanyl system. In particular, a monolithic fentanyl patch according to the invention produces substantially the same pharmacokinetic effects (as measured by the area under the blood or plasma drug concentration-time curve (AUC) and the peak plasma concentration (Cₘₐₓ) of the drug) as compared to the DURAGESIC^{®} transdermal fentanyl system, when studied under similar experimental conditions, as described in greater detail hereinafter.

In additional preferred embodiments, a patch of this invention is pharmacologically equivalent to the DURAGESIC^{®} fentanyl system. In particular, a monolithic sufentanil patch according to the invention produces substantially the same therapeutic effects as compared to the DURAGESIC^{®} transdermal fentanyl system, when studied under similar experimental conditions, as described in greater detail hereinafter.

In general, the standard bioequivalence study is conducted in a crossover fashion in a small number of volunteers, usually with 24 to 36 healthy normal adults. Single doses of the drug containing test product, e.g., transdermal fentanyl patch according to the invention, and reference product, e.g., DURAGESIC^{®} fentanyl system, are administered and blood or plasma levels of the drug are measured over time. Characteristics of these concentration-time curves, such as the area under the blood or plasma drug concentration-time curve (AUC) and the peak blood or plasma concentration (Cₘₐₓ) of the drug, are examined by statistical procedures as described in greater detail hereinafter. In general, two one-sided statistical tests are carried out using the log-transformed parameter (AUC and Cₘₐₓ) from the bioequivalence study. The two one-sided tests are carried out at the 0.05 level of significance and the 90% confidence interval is computed. The test and the reference formulation/composition are considered bioequivalent if the confidence interval around the ratio of the mean (test/reference product) value for a pharmacokinetic parameter is no less than 80% on the lower end and no more than 125% on the upper end.

Two different products are generally considered to be "pharmacologically equivalent" if they produce substantially the same therapeutic effects when studied under similar experimental conditions, as demonstrated through several in vivo and in vitro methods as described above. Therapeutic effects depend on various factors, such as, potency of the drug, the solubility and diffusivity of the drug in the skin, thickness of the skin, concentration of the drug within the skin application site, concentration of the drug in the drug reservoir, and the like, as described in greater detail hereinafter. In general, pharmacological equivalence is demonstrated using measures such as the peak blood or plasma concentration of the drug normalized for the rate of drug administered (i.e. normalized Cₘₐₓ as defined above) and the peak blood or plasma concentration of the drug standardized per unit area of the active drug delivery area of the system (i.e. standardized Cₘₐₓ as defined above).

When comparing two different products whose drug administration rate is proportional to the size of the patch, the is no difference if the peak blood or plasma concentration of the drug (Cₘₐₓ) is normalized for the rate of drug administered, or standardized per unit area of the active drug delivery area of the system, in order to establish bioequivalence or pharmacological equivalence. However, when comparing two different products having different drug administration rate per unit area, it is necessary to normalize the peak blood or plasma concentration of the drug (Cₘₐₓ) on the basis of the rate of drug administered to establish bioequivalence or pharmacological equivalence.

### Methods of Manufacture

The transdermal devices are manufactured according to known methodology. A solution of the polymeric reservoir material, as described above, is added to a double planetary mixer, followed by addition of desired amounts of the drug, preferably fentanyl or sufentanil, more preferably fentanyl base or sufentanil base, and optionally, a permeation enhancer. Preferably, the polymeric reservoir material is solubilized in an organic solvent, e.g., ethanol, ethyl acetate, hexane, and the like. The mixer is then closed and activated for a period of time to achieve acceptable uniformity of the ingredients. The mixer is attached by means of connectors to a suitable casting die located at one end of a casting/film drying line. The mixer is pressurized using nitrogen to feed solution to the casting die. Solution is cast as a wet film onto a moving siliconized polyester web. The web is drawn through the lines and a series of ovens are used to evaporate the casting solvent to acceptable residual limits. The dried reservoir film is then laminated to a selected backing membrane and the laminate is wound onto the take-up rolls. In subsequent operations, individual transdermal patches are die-cut, separated and unit-packaged using suitable pouchstock. Patches are cartoned using conventional equipment. In another process, the drug reservoir can be formed using dry-blending and thermal film-forming using equipment known in the art. Preferably, the materials are dry blended and extruded using a slot die followed by calendering to an appropriate thickness.

### Experimental

Below are examples of specific embodiments for carrying out the present invention. The examples are offered for illustrative purposes only, and are not intended to limit the scope of the present invention in any way.

Efforts have been made to ensure accuracy with respect to numbers used (e.g., amounts, temperatures, etc.), but some experimental error and deviation should, of course, be allowed for.

Specific examples of various transdermal patches of the invention which are capable of administering fentanyl and analogs thereof for extended periods of time will be described in the examples set for hereinafter. The adhesive-reservoir patches wherein the reservoir comprises a single phase polymeric composition of free undissolved components containing an amount of fentanyl or sufentanil at subsaturation concentration are presently considered preferable according to our invention. In the following examples all percentages are by weight unless noted otherwise.

### EXAMPLE 1

Monolithic transdermal patches according to Figure 1 were prepared in 5.5, 11, 22, 33 and 44 cm² sizes comprising respectively, 2.2, 4.4, 8.8, 13.2 and 17.6 mg each of fentanyl base.

A polacrylate adhesive (National Starch 87-2287, 100 g) was solubilized in a solvent (ethyl acetate, 128 ml). Fentanyl base was added to the polacrylate adhesive solution in amounts sufficient to generate a mixture containing 3.4 wt% of fentanyl in the adhesive solution and stirred to dissolve the drug. The solution was cast into a 2 mil thick reservoir layer and the solvent was evaporated. After solvent evaporation, a 3 mil thick backing layer comprised of a multilaminate of nonlinear LDPE layer/linear LDPE layer/nonlinear LDPE layer was laminated on to the adhesive drug reservoir layer using standard procedures. Individual patches were die-cut from this laminate in 5.5, 11, 22, 33 and 44 cm² sizes comprising respectively, 2.2, 4.4, 8.8, 13.2 and 17.6 mg each of fentanyl, to generate monolithic transdermal patches containing 0.4 mg/cm² of fentanyl base.

### EXAMPLE 2

Monolithic transdermal patches according to Figure 1 were prepared in 5.5, 11, 22, 33 and 44 cm² sizes comprising respectively, 2.2, 4.4, 8.8, 13.2 and 17.6 mg each of fentanyl base.

A polacrylate adhesive (National Starch 87-4287, 100 g) was solubilized in a solvent (ethyl acetate, 160 ml). Fentanyl base was added to the polacrylate adhesive solution in amounts sufficient to generate a mixture containing 2.8 wt% of fentanyl in the adhesive solution and stirred to dissolve the drug. The solution was cast into a 2 mil thick reservoir layer and the solvent was evaporated. After solvent evaporation, a 1.7 mil thick backing layer comprised of a multilaminate of polyethylene/polyurethane/ polyester layer was laminated on to the adhesive drug reservoir layer using standard procedures. Individual patches were die-cut from this laminate in 5.5, 11, 22, 33 and 44 cm² sizes comprising respectively, 2.2, 4.4, 8.8, 13.2 and 17.6 mg each of fentanyl, to generate monolithic transdermal patches containing 0.4 mg/cm² of fentanyl base.

### EXAMPLE 3

Monolithic transdermal patches were prepared in 5.5, 11, 22, 33 and 44 cm² sizes comprising 2.2, 4.4, 8.8, 13.2 and 17.6 mg of fentanyl, respectively, as described in Examples 1 and 2 with the following exceptions. Materials were dry blended, in the absence of ethyl acetate, and extruded using a slot die followed by calendering to an appropriate thickness.

### EXAMPLE 4

Monolithic transdermal patches according to Figure 1 were prepared in 5.2, 10.5, 21, 31.5 and 42 cm² sizes comprising respectively, 2, 4, 8, 12 and 16 mg each of fentanyl base. A polacrylate adhesive (National Starch 87-2287, 500 g) and glyceryl monolaurate (GML, 10 g) were dissolved in a solvent (ethyl acetate, 640 ml). Fentanyl base was added to the polacrylate adhesive solution in amounts sufficient to generate a mixture containing 4 wt% of fentanyl in the adhesive solution and stirred to dissolve the drug. The solution was cast into a 1.8 mil thick reservoir layer, and the solvent was evaporated. After solvent evaporation, a 3 mil thick backing layer comprised of a multilaminate of nonlinear LDPE layer/linear LDPE layer/nonlinear LDPE layer was laminated on to the adhesive drug reservoir layer using standard procedures. Individual patches were die-cut from this laminate in 5.2, 10.5, 21, 31.5 and 42 cm² sizes comprising respectively, 2, 4, 8, 12 and 16 mg each of fentanyl, to generate monolithic transdermal patches containing 0.35 mg/cm² of fentanyl base.

### EXAMPLE 5

Monolithic transdermal patches were prepared in 5.2, 10.5, 21, 31.5 and 42 cm² sizes comprising respectively, 2, 4, 8, 12 and 16 mg each of fentanyl, as described in Example 4 with the following exceptions. Materials were dry blended, in the absence of ethyl acetate, and extruded using a slot die followed by calendering to an appropriate thickness.

### EXAMPLE 6

Monolithic transdermal patches were prepared in 2.54 cm² sizes comprising respectively, 0.25, 0.5, 0.75, 1.0 and 1.1 mg (corresponding to 2, 4, 6, 8 and 9 wt% respectively) each of sufentanil, and a polacrylate adhesive (National Starch 87-4287, as described in Examples 1 and 2, above.

### EXAMPLE 7

Monolithic transdermal systems were prepared in 2.54 cm² sizes comprising 1.1 mg of sufentanil and a permeation enhancer, each system respectively comprising one of: lauryl pyroglutamate (1.1 mg, 9 wt%), glycerol monocaprylate (1.2 mg, 10 wt%), and glycerol monocaprate (0.625 mg, 5 wt%), as described in Example 6.

Similarly, monolithic transdermal systems comprising respectively, 0.25, 0.5, 0.75 and 1.0 mg (corresponding to 2, 4, 6 and 8 wt% respectively) each of sufentanil, and a permeation enhancer are prepared as described above.

### EXAMPLE 8

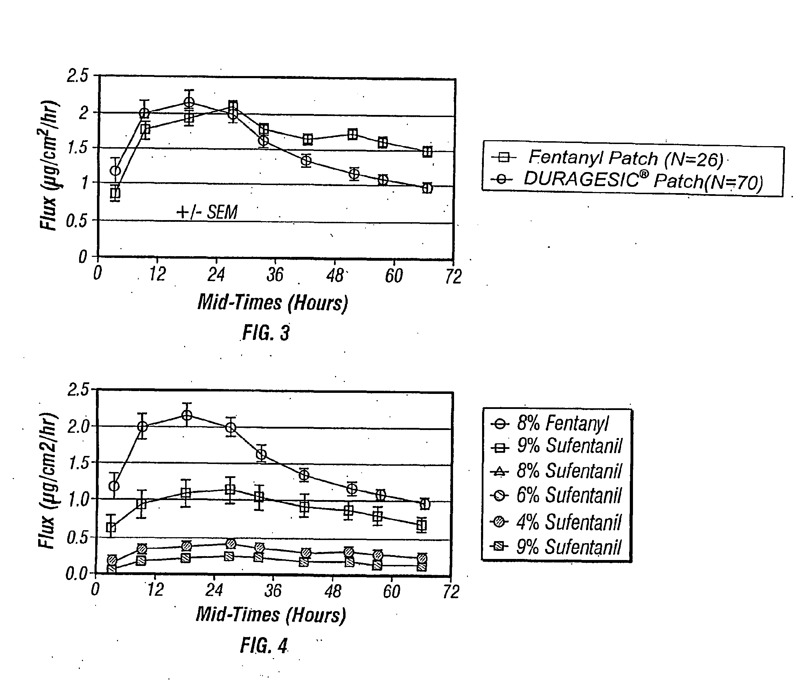

The in vitro fentanyl flux studies were conducted using various transdermal fentanyl patches - monolithic fentanyl patches and DURAGESIC^{®} fentanyl system. The monolithic fentanyl patches containing 0.4 mg/cm² of fentanyl base for a 2.54 cm² patch were prepared as described in Example 1. The comparative transdermal flux is illustrated in Figure 3. The in vitro fentanyl flux studies were conducted using a two-compartment diffusion cell with a section of human cadaver epidermis mounted between the cell halves. A transdermal patch was adhered to one side of the skin and a drug-receiving medium was placed on the receptor-side of the cell. The apparatus was placed in a water bath maintained at 32 ± 0.3° C. Samples of the receptor medium were collected over a period of 72 hours for HPLC analysis of drug concentration. From a knowledge of the receptor volume, the area of skin exposure, the time interval between samplings and the drug concentration, the rate of fentanyl transport was calculated. The time averaged rate of drug permeation was approximately 1.5 (±20% RSD) µg/h-cm², which was a mean value of at least four experiments using at least four separate skin donors in triplicate (i.e. n = 12).

As illustrated in Figure 3, the drug flux from the non-rate controlled, monolithic, subsaturated patch of the invention is greater than the drug flux from the rate controlled, liquid reservoir, DURAGESIC^{®} fentanyl depot patch up to 24 hours. From 24 hours up to 72 hours, the drug flux from the non-rate controlled, monolithic, subsaturated patch of the invention decreases as compared to the drug flux from the rate controlled, liquid reservoir, DURAGESIC^{®} fentanyl depot patch.

### EXAMPLE 9

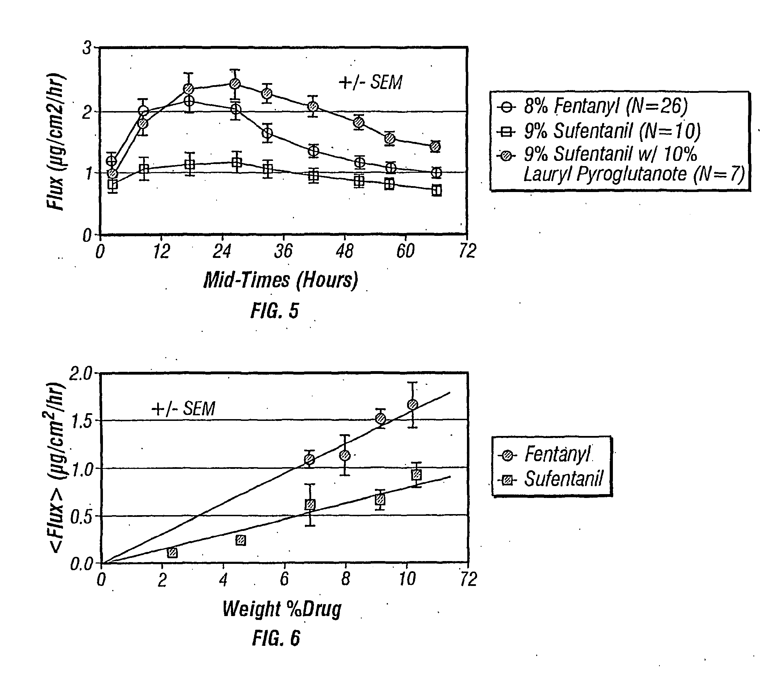

The in vitro fentanyl flux studies were conducted as described in Example 8 using various monolithic fentanyl and sufentanil patches. The monolithic fentanyl patches containing 0.4 mg/cm² of fentanyl base and 0.25, 0.5, 0.75, 1.0 and 1.1 mg/cm² (corresponding to 2, 4, 6, 8 and 9 wt% respectively) each of sufentanil for a 2.54 cm² patch were prepared as described in Examples 1-7. The comparative transdermal flux is illustrated in Figures 4, 5 and 6.

### EXAMPLE 10

The in vivo fentanyl flux studies were conducted using various transdermal fentanyl patches - monolithic fentanyl patches as described in Example 1, and DURAGESIC^{®} fentanyl system, and the comparative pharmacokinetic parameters are tabulated in Table 1 and 2 below. The pharmacokinetic parameters of the patches were evaluated as follows.

The study was a single center, randomized, single-dose, open label, eight-sequence, eight-treatment, three-period crossover study. Healthy adult subjects were randomly assigned to one of 8 treatment sequences. There was a minimum washout period of at least 72 hours and not more than 14 days between treatment arms. The washout period began upon removal of the study systems. Each subject received naltrexone 14 hours before system application and twice daily during application. The system was removed 72 hours after application. Serial blood samples were collected from each subject during each treatment at pre-dose and 0.5, 1, 2, 3, 5, 8, 12, 18, 24, 30, 36, 42, 48, 54, 60, 66, 72, 73, 74, 78, 84, and 96 hours post dose. Blood samples were analyzed using radioimmunoassay for fentanyl concentration levels.

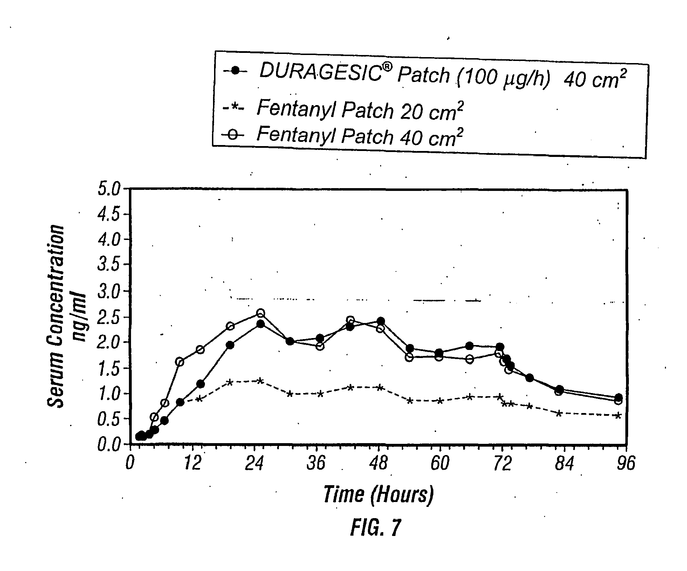

The results of the in vivo study are tabulated in Tables 1 and 2. Figure 7 illustrates serum fentanyl concentrations following transdermal application of various fentanyl patches - one application of fentanyl patch (20 cm²); two applications of fentanyl patch (40 cm²), and DURAGESIC^{®} fentanyl system (100 µg/h, 40 cm²), up to 96 hours after first administration.

**TABLE 1**

| Comparative Pharmacokinetic (PK) Parameters for fentanyl patches and DURAGESIC^{®} fentanyl system | | | | | |
|---|---|---|---|---|---|
| Dose (µg/h) | Size (cm²) | Fentanyl content (mg) | Cₘₐₓ (ng/ml) | Standardized Cₘₐₓ (ng/ml-cm²) | Normalized Cₘₐₓ (ng/ml-(mg/h)) |
| DURAGESIC^{®} | | | | | |
| 25 | 10 | 2.5 | 0.6 | 0.06 | 24 |
| 50 | 20 | 5.0 | 1.4 | 0.07 | 28 |
| 75 | 30 | 7.5 | 1.7 | 0.05 | 22.7 |
| 100 | 40 | 10.0 | 2.5 | 0.06 | 25 |

| Transdermal fentanyl patches | | | | | |
|---|---|---|---|---|---|
| 12.5 | 5.5 | 2.2 | 0.33 | 0.06 | 26.4 |
| 25 | 11 | 4.4 | 0.66 | 0.06 | 26.4 |
| 50 | 22 | 8.8 | 1.32 | 0.06 | 26.4 |
| 75 | 33 | 13.2 | 1.98 | 0.06 | 26.4 |
| 100 | 44 | 17.6 | 2.64 | 0.06 | 26.4 |

**Table 2**

| Mean (CV%^{a}) Pharmacokinetic (PK) Parameters for Transdermal Fentanyl Patches | | | |
|---|---|---|---|
| PK parameters | DURAGESIC^{®} fentanyl patch (100 µg/h), 40 cm² (n=36) | Fentanyl patch^{b} 20 cm² (n=20) | Fentanyl patch^{c} 40 cm² (n=19) |
| Cmax (ng/mL) | 2.76 (36.0) | 1.32 (44.5) | 2.91 (61.0) |
| Tmax (h) | 41.89 (44.93) | 30.10 (61.60) | 31.37 (54.93) |
| AUC₀₋₉₆ (ng.h/mL) | 148.5 (36.3) | 73.1 (40.6) | 154.6 (42.9) |
| AUC_{inf}(ng.h/mL) | 172.7 (38.6) | 85.1 (42.8) | 166.9 (41.2) |
| Half-life (h) | 20.3 (39.8) | 21.1 (29.6) | 20.1 (42.6) |
| Flux Rate (µg/cm²/h) | 2.56 (12.9) | 2.99 (17.8) | 2.94 (19.1) |

| | | | |
|---|---|---|---|
| a = percent coefficient of variation b = one application of a 20 cm² patch c = two applications of 20 cm² patches | | | |

### EXAMPLE 11

The in vivo fentanyl flux studies were conducted using various transdermal fentanyl patches - monolithic fentanyl patches as described in Example 1, and DURAGESIC^{®} fentanyl system, as described in Example 9 with the following exceptions.

The study was a single center, randomized, single-dose, open label, two-sequence, two-treatment, two-period crossover study. Healthy adult subjects were randomly assigned to one of two treatment sequences. There was a minimum washout period of at least 72 hours and not more than 14 days between treatment arms. The washout period began upon removal of the study systems. Each subject received naltrexone 14 hours before system application and twice daily during application. The system was removed 72 hours after application. Serial blood samples were collected from each subject during each treatment at pre-dose and 0.5, 1, 2, 3, 5, 8, 12, 18, 24, 30, 36, 42, 48, 54, 60, 66, 72, 73, 74, 78, 84, 96, 108 and 120 hours post dose. Blood samples were analyzed using radioimmunoassay for fentanyl concentration levels.

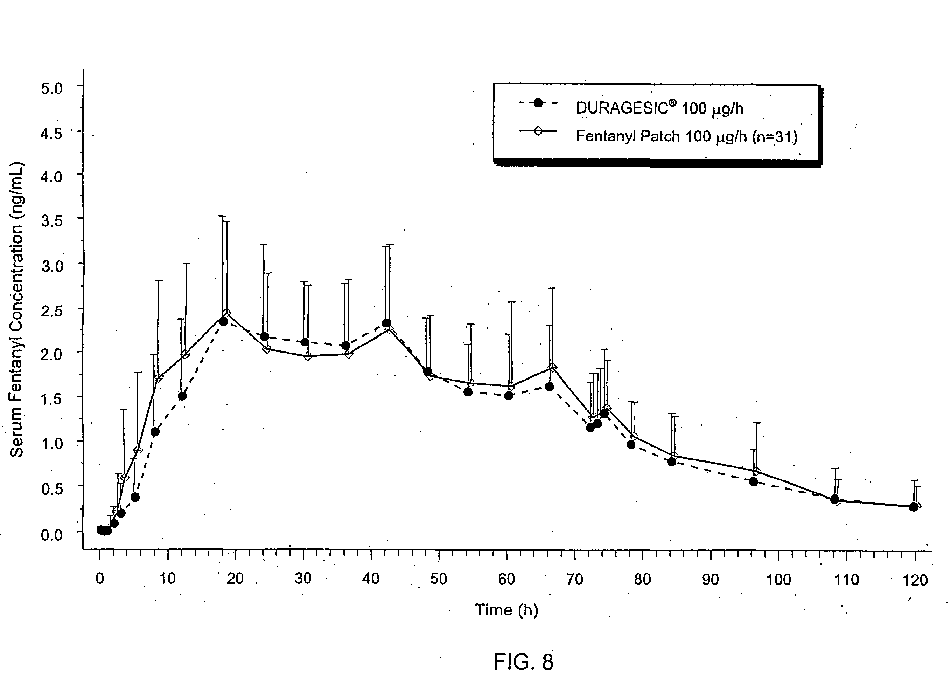

The results of the in vivo study are tabulated in Table 3. Figure 8 illustrates serum fentanyl concentrations following transdermal application of various fentanyl patches - a fentanyl patch of the invention (100 µg/h, 40 cm²), and a DURAGESIC^{®} fentanyl system (100 µg/h, 40 cm²), up to 120 hours after first administration. Characteristics of these concentration-time curves, such as the area under the serum drug concentration-time curve (AUC) and the peak blood or plasma concentration (Cₘₐₓ) of the drug, were examined by statistical procedures as described earlier. Two one-sided statistical tests were carried out using the log-transformed parameter (AUC and Cₘₐₓ) from the in vivo (bioequivalence) study. The two one-sided tests were carried out at the 0.05 level of significance and the 90% confidence interval was computed. The test and the reference formulation/composition were considered bioequivalent if the confidence interval around the ratio of the mean (test/reference product i.e. Treatment B/Treatment A) value for a pharmacokinetic parameter is no less than 80% on the lower end and no more than 125% on the upper end. The results of the statistical analysis of log transformed pharmacokinetic (PK) parameters are tabulated in Table 4.

**Table 3**

| Mean (CV%^{a}) Pharmacokinetic (PK) Parameters for Transdermal Fentanyl Patches | | |
|---|---|---|
| PK Parameter | DURAGESIC® Fentanyl Patch (100 µg/h) 40 cm² (n=33) | Fentanyl Patch (100 µg/h) 40 cm² (n=31) |
| Cmax (ng/mL) | 2.86 (39.6) | 2.93 (40.7) |
| Tmax (h) | 32.2 (49.7) | 29.4 (67.4) |
| AUC₀₋₁₂₀ (ng.h/mL) | 145.9 (38.1) | 154.6 (40.3) |
| AUC_{inf} (ng.h/mL) | 159.7 (35.0) | 166.8 (37.2) |
| Half-life (h) | 21.2 (28.6) | 21.3 (35.3) |

| | | |
|---|---|---|
| a = percent coefficient of variation | | |

**Table 4**

| Statistical Analysis of Log Transformed Pharmacokinetic (PK) Parameters | | |
|---|---|---|
| Parameter | LnAUC_{inf} | LnCₘₐₓ |
| Contrast^{a} | Treatment B/Treatment A | Treatment B/Treatment A |
| N | 30 | 30 |
| Ratio (%) | 106.58 | 98.46 |
| P value | 0.068 | 0.808 |
| Power^{b} | >99 | 92.4 |
| 90% Conf. Interval | | |
| Lower | 100.67 | 88.39 |
| Upper | 112.84 | 109.67 |

| | | |
|---|---|---|
| ^{a} Treatment A = DURAGESIC® fentanyl patch (100 µg/h) Treatment B = Fentanyl patch (100 µg/h) ^{b} The power to detect a difference equal to 20% of the reference mean, at a significance level of 0.05, expressed as a percentage of the reference mean. The reference is the second treatment appearing in each contrast. | | |

Thus, as evidenced from the results tabulated above and illustrated in Figures 3-8, the monolithic, subsaturated, transdermal patch of the present invention comprising a drug reservoir comprising a single phase polymeric composition comprising a subsaturation concentration of the drug, are bioequivalent products to the rate-controlled, saturated DURAGESIC^{®} fentanyl system. In particular, the monolithic subsaturated patches according to the invention display pharmacokinetic parameters comparable to the transdermal DURAGESIC^{®} fentanyl system.

The present invention is described and characterized by one or more of the following features and/or characteristics, either alone or in combination with one or more of the other features and characteristics:

A transdermal patch for administering fentanyl or an analog thereof through the skin comprising: (a) a backing layer; (b) a reservoir disposed on the backing layer, at least the skin contacting surface of said reservoir being adhesive; said reservoir comprising a single phase polymeric composition free of undissolved components containing an amount of fentanyl or an analog thereof sufficient to induce and maintain analgesia in a human for at least three days and up to seven days; the patch exhibits the patch exhibits a normalized Cₘₐₓ ranging from 3.3 to 82.5 ng/ml-(mg/h) and a standardized Cₘₐₓ of 0.001 to 0.2 ng/ml-cm² and a steady state drug flux of 0.1 to 20 µg/cm²/hr. The reservoir is formed from a polyacrylate adhesive. The reservoir comprises a drug selected from the group consisting of fentanyl, alfentanil, iofentanil, remifentanil, sufentanil and trefentanil. Preferably, the drug is in the base form, and the preferred drug is fentanyl or sufentanil. The drug reservoir comprises a polymer having a solubility for fentanyl and analogs thereof of 1 wt% to 25 wt%; 0.05 to 1.75 mg/cm² of fentanyl or analogs thereof; and has a thickness of 0.0125 mm (0.5 mil) to 0.1mm (4 mil). The reservoir optionally comprises a permeation enhancer. The patch comprises a backing layer comprising a polymer selected from the group consisting of polyurethane, polyvinyl acetate, polyvinylidene chloride, polyethylene, polyethylene terephthalate (PET), PET-polyolefin laminates, and polybutylene terephthalate, preferably low density polyethylene (LDPE) materials; wherein the backing layer has a thickness of 2 mil to 5 mil. Preferably, the drug is in the base form and the preferred drug is fentanyl, wherein fentanyl, has a solubility of 7 wt% to 12 wt% in the reservoir; the reservoir is formed from a polyacrylate adhesive, more preferably a polyacrylate adhesive having a T_{g} less than -10 °C. In preferred embodiments, the reservoir comprises 0.05 to 1.75 mg/cm² of fentanyl base; preferably 0.07 to 1.50 mg/cm² of fentanyl base; preferably ; 0.08 to 1.25 mg/cm² of fentanyl base; more preferably 0.09 to 1.0mg/cm² of fentanyl base; more preferably 0.1 to 0.76 mg/cm² of fentanyl base; and even more preferably 0.12 to 0.5 mg/cm² of fentanyl base. In alternative preferred embodiments, the drug is in the base form and the preferred drug is sufentanil, wherein sufentanil has a solubility of 1 wt% to 25 wt% In the reservoir; the reservoir Is formed from an a polyacrylate adhesive, preferably a polyacrylate adhesive having a T_{g} less than -10°C. In preferred embodiments, the reservoir comprises 0.05 to 1.75 mg/cm² of sufentanil base; preferably 0.07 to 1.50 mg/cm² of sufentanil base; preferably 0.08 to 1.25 mg/cm² of sufentanil base; preferably 0.09 to 1.0 mg/cm² of sufentanil base; more preferably 0.1 to 0.75 mg/cm² of sufentanil base; more preferably 0.12 to 0.5 mg/cm² of sufentanil base; and even more preferably 0.25 to 0.4 mg/cm² of sufentanil base.

A transdermal patch for administering fentanyl and analogs thereof through the skin comprising (a) a backing layer; (b) a reservoir disposed on the backing layer, at least the skin contacting surface of said reservoir being adhesive; said reservoir comprising a single phase polymeric composition free of undissolved components containing an amount of fentanyl or an analog thereof sufficient to induce and maintain analgesia in a human for at least three days; wherein the patch is bioequivalent or pharmacologically equivalent to DURAGESIC^{®} transdermal fentanyl system; the patch exhibits a normalized Cₘₐₓ ranging from 3.3 to 82.5 ng/ml-(mglh) and a standardized Cₘₐₓ of 0.001 to 0.2 ng/ml-cm² and a steady state drug flux of 0.1 to 20 µg/cm²/hr. Preferably, the drug is in the base form and the preferred drug is fentanyl, wherein fentanyl has a solubility of 7 wt% to 12 wt% in the reservoir; the reservoir is formed from a polyacrylate adhesive, preferably a polyacrylate adhesive having a Tg less than -10°C. In preferred embodiments, the reservoir comprises 0.05 to 1.75 mg/cm² of fentanyl base; preferably 0.07 to 1.50 mg/cm² of fentanyl base; preferably 0.08 to 1.25 mg/cm² of fentanyl base; more preferably 0.09 to 1.0 mg/cm² of fentanyl base; more preferably 0.1 to 0.75 mg/cm² of fentanyl base; and even more preferably 0.12 to 0.5 mg/cm² of fentanyl base. In alternative preferred embodiments, the drug is in the base form and the preferred drug is sufentanil, wherein sufentanil has a solubility of 1 wt% to 25 wt% in the reservoir; the reservoir is formed from a polyacrylate adhesive, preferably a polyacrylate adhesive having a T_{g} less than -10°C. In preferred embodiments, the reservoir comprises 0.05 to 1.75 mg/cm² of sufentanil base; preferably 0.07 to 1.50 mg/cm² of sufentanil base; preferably 0.08 to 1.25 mg/cm² of sufentanil base; more preferably 0.09 to 1.0 mg/cm² of sufentanil base; more preferably 0.1 to 0.75 mg/cm² of sufentanil base; more preferably 0.12.to 0.5 mg/cm² of sufentanil base; and even more preferably 0.25 to 0.4 mg/cm² of sufentanil base.

A monolithic transdermal patch for administering fentanyl, comprising an adhesive fentanyl reservoir on a backing layer, said reservoir comprising a single phase polymeric composition free of undissolved components containing a polyacrylate adhesive having sufficient solubility for fentanyl to contain dissolved fentanyl in an amount sufficient to induce and maintain analgesia in a human for at least three days and up to seven days, wherein fentanyl has a solubility of at least 4 wt% in said reservoir, the reservoir has a thickness of 0.0125 mm (0.5 mil) to 0.1 mm (4 mil); the patch being completely free from a rate controlling membrane, the patch exhibiting a normalized Cₘₐₓ ranging from 3.3 to - 82.5 ng/ml-(mg/h); and a standardized Cₘₐₓ of 0.01 to 0.2 ng/ml-cm² and a steady state drug flux of about 1-10 µg/cm²/hr; and wherein the patch is bioequivalent to DURAGESIC^{®} transdermal fentanyl system.

A monolithic transdermal patch for administering sufentanil, comprising an adhesive sufentanil reservoir on a backing layer, said reservoir comprising a single phase polymeric composition free of undissolved components containing a polyacrylate adhesive having sufficient solubility for sufentanil to contain dissolved sufentanil in an amount sufficient to induce and maintain analgesia in a human for at least three days and up to seven days, wherein sufentanil has a solubility of at least 5 wt% in said reservoir; the reservoir has a thickness of 0.0125 mm (0.5 mil) to 0.1 mm (4 mil); the patch being completely free from a rate controlling membrane, the patch exhibiting a normalized Cₘₐₓ ranging from 0.04 to 10 ng/ml-(mg/h); and a standardized Cₘₐₓ of 0.001 to 0.0.05 ng/ml-cm² and a steady state drug flux of about 1 to about 10 µg/cm²/hr; and wherein the patch is pharmacologically equivalent to DURAGESIC^{®} transdermal fentanyl system.

## Claims

1. A monolithic transdermal patch for administering fentanyl, alfentanil, carfentanil, lofentanil, remifentanil, sufentanil or trefentanil through the skin comprising:
(a) a backing layer;
(b) a reservoir disposed on the backing layer, at least the skin contacting surface of said reservoir being adhesive; said reservoir comprising a single phase polymeric composition free of undissolved components containing an amount of a drug selected from the group consisting of fentanyl, alfentanil, carfentanil, lofentanil, remifentanil, sufentanil and trefentanil sufficient to induce and maintain analgesia in a human for at least three days;
**characterised in that** the reservoir is formed from a polyacrylate adhesive and has a thickness of 0.0125 mm (0.5 mil) to 0.1 mm (4 mil).

2. The patch of claim 1, which contains fentanyl,

3. The patch of claim 1, which contains alfentanil, lofentanil, remifentanil or sufentanil as the drug.

4. The patch of claim 3 wherein the drug is sufentanil.

5. The patch of any preceding claim wherein said polyacrylate adhesive has a Tg less than -10°C.

6. The patch of any one of claims 1 to 5 wherein said patch exhibits a normalized Cₘₐₓ of 3.3 to 82.5 ng/ml-(mg/h).

7. The patch of claim 6 wherein the patch exhibits a steady state drug flux of 0.1 to 20µg/cm²/hr, preferably 1 to 10µg/cm²/hr.

8. The patch of any one of claims 1 to 7 wherein said patch exhibits a standardized Cₘₐₓ of 0.001 to 0.2ng/ml-cm.

9. The patch of any one of claims 1 to 8 wherein said reservoir comprises an amount of the dissolved drug sufficient to induce and maintain analgesia for 3-7 days.

10. The patch of claim 9 wherein said reservoir comprises a polymer having a solubility for the drug of 1 wt% to 25 wt%, preferably 7 wt% to 12 wt%.

11. The patch of claim 9 or claim 10 wherein the reservoir comprises 0.05 to 1.75 mg/cm², preferably 0.08 to 1.25 mg/cm², more preferably 0.1 to 0.75 mg/cm², most preferably 0.12 to 0.5 mg/cm² of the drug.

12. The patch of any one of claims 1 to 13 wherein the reservoir further comprises a permeation enhancer.

13. The patch of any one of claims 1 to 12 wherein the backing layer comprises a polyurethane, polyvinyl acetate, polyvinylidene chloride, polyethylene, polyethylene terephthalate (PET), PET-polyolefin laminate or a polybutylene terephthalate polymer.

14. The patch of claim 13, wherein the backing layer comprises a low density polyethylene (LDPE) material, a medium density polyethlene (MDPE) material or a high density polyethylene (HDPE) material.

15. The patch of claim 14, wherein the backing layer comprises a low density polyethylene (LDPE) material.

16. The patch of any one of claims 13 to 15 wherein the backing layer has a thickness of 0.012 mm (0.5 mil) to 0.125 mm (5 mil).

17. A transdermal patch according to any one of claims 1 to 16, for administering fentanyl, comprising an adhesive fentanyl reservoir on a backing layer; said reservoir comprising a single phase polymeric composition free of undissolved components containing a polyacrylate adhesive having sufficient solubility for fentanyl to contain dissolved fentanyl in an amount sufficient to induce and maintain analgesia in a human for at least three days, said patch exhibiting a normalized Cₘₐₓ of 3.3 to 82.5 ng/ml-(mg/h).

18. A transdermal patch according to any one of claims 1 to 16, for administering fentanyl, comprising an adhesive fentanyl reservoir on a backing layer; said reservoir comprising a single phase polymeric composition free of undissolved components containing a polyacrylate adhesive having sufficient solubility for fentanyl to contain dissolved fentanyl in an amount sufficient to induce and maintain analgesia in a human for at least three days, said patch exhibiting a standardized Cₘₐₓ of 0.01 to 0.2 ng/ml-cm².

19. A transdermal patch according to any one of claims 1 to 16, for administering sufentanil, comprising an adhesive sufentanil reservoir on a backing layer; said reservoir comprising a single phase polymeric composition free of undissolved components containing a polyacrylate adhesive having sufficient solubility for sufentanil to contain dissolved sufentanil in an amount sufficient to induce and maintain analgesia in a human for at least three days, said patch exhibiting a normalized Cₘₐₓ of 0.04 to 10 ng/ml-(mg/h).

20. A transdermal patch according to any one of claims 1 to 16, for administering sufentanil, comprising an adhesive sufentanil reservoir on a backing layer; said reservoir comprising a single phase polymeric composition free of undissolved components containing a polyacrylate adhesive having sufficient solubility for sufentanil to contain dissolved sufentanil in an amount sufficient to induce and maintain analgesia in a human for at least three days, said patch exhibiting a standardized Cₘₐₓ of 0.001 to 0.05 ng/ml-cm².

21. The patch of any one of claims 1 to 20 which is completely free from a rate controlling membrane.

## Patentansprüche

1. Monolithisches transdermales Pflaster zur Verabreichung von Fentanyl, Alfentanil, Carfentanil, Lofentanil, Remifentanil, Sufentanil oder Trefentanil durch die Haut, umfassend:
(a) eine Trägerlage;
(b) einen Vorrat, der auf der Trägerlage angeordnet ist, wobei zumindest die mit der Haut in Kontakt kommende Oberfläche des Vorrats klebrig ist; wobei der Vorrat eine einphasige polymerische Zusammensetzug frei von ungelösten Komponenten umfasst, die eine Menge eines Wirkstoffs ausgewählt aus der Gruppe bestehend aus Fentanyl, Alfentanil, Carfentanil, Lofentanil, Remifentanil, Sufentanil oder Trefentanil enthält, die ausreichend ist, um eine Schmerzfreiheit in einem Menschen für mindestens drei Tage zu induzieren und aufrechtzuerhalten;
**dadurch gekennzeichnet, dass** der Vorrat aus einem Polyacrylat-Kleber geformt ist und eine Dicke von 0,0125 mm (0,5 mil) bis 0,1 mm (4 mil) aufweist.

2. Pflaster nach Anspruch 1, das Fentanyl enthält.

3. Pflaster nach Anspruch 1, das Alfentanil, Lofentanil, Remifentanil oder Sufentanil als den Wirkstoff enthält.

4. Pflaster nach Anspruch 3, wobei der Wirkstoff Sufentanil ist.

5. Pflaster nach einem der voranstehenden Ansprüche, wobei der Polyacrylat-Kleber eine Tg von weniger als -10°C aufweist.

6. Pflaster nach einem der Ansprüche 1 bis 5, wobei das Pflaster eine normalisierte Cₘₐₓ von 3,3 bis 82,5 ng/ml-(mg/h) aufweist.

7. Pflaster nach Anspruch 6, wobei das Pflaster einen Steady-State Wirkstofffluss von 0,1 bis 20 µg/cm²/h, bevorzugt 1 bis 10 µg/cm²/h aufweist.

8. Pflaster nach einem der Ansprüche 1 bis 7, wobei das Pflaster eine standardisierte Cₘₐₓ von 0,001 bis 0,2 ng/ml-cm aufweist.

9. Pflaster nach einem der Ansprüche 1 bis 8, wobei der Vorrat eine Menge des gelösten Wirkstoffs umfasst, die ausreichend ist, um eine Schmerzfreiheit in einem Menschen für 3 bis 7 Tage zu induzieren und aufrechtzuerhalten.

10. Pflaster nach Anspruch 9, wobei der Vorrat ein Polymer umfasst, das eine Löslichkeit für den Wirkstoff von 1 Gew.-% bis 25 Gew.-%, bevorzugt 7 Gew.-% bis 12 Gew.-%, aufweist

11. Pflaster nach Anspruch 9 oder Anspruch 10, wobei der Vorrat 0,05 bis 1,75 mg/cm², bevorzugt 0,08 bis 1,25 mg/cm², weiter bevorzugt 0,1 to 0,75 mg/cm², am meisten bevorzugt 0,12 to 0,5 mg/cm² des Wirkstoffs umfasst.

12. Pflaster nach einem der Ansprüche 1 bis 13, wobei der Vorrat weiterhin einen Permeationsverstärker umfasst.

13. Pflaster nach einem der Ansprüche 1 bis 12, wobei die Trägerlage ein Polyurethan, Polyvinylacetat, Polyvinylidenchlorid, Polyethylen, Polyethylenterephthalat (PET), PET-Polyolefinlaminat oder ein Polybutylenterephthalat-Polymer umfasst.

14. Pflaster nach Anspruch 13, wobei die Trägerlage ein Polyethylen-Material geringer Dichte (LDPE), ein Polyethylen-Material mittlerer Dichte (MDPE) oder ein Polyethylen-Material hoher Dichte (HDPE) umfasst.

15. Pflaster nach Anspruch 14, wobei die Trägerlage ein Polyethylen-Material geringer Dichte (LDPE) umfasst.

16. Pflaster nach einem der Ansprüche 13 bis 15, wobei die Trägerlage eine Dicke von 0,012 mm (0,5 mil) bis 0,125 mm (5 mil) aufweist.

17. Transdermales Pflaster nach einem der Ansprüche 1 bis 16 zur Verabreichung von Fentanyl umfassend einen Kleber-Fentanylvorrat auf einer Trägerlage; wobei der Vorrat eine einphasige polymerische Zusammensetzung frei von ungelösten Komponenten umfasst, enthaltend einen Polyacrylat-Kleber der eine ausreichende Löslichkeit für Fentanyl aufweist um gelöstes Fentanyl in einer Menge zu enthalten, die ausreichend ist, um eine Schmerzfreiheit in einem Menschen für mindestens drei Tage zu induzieren und aufrechtzuerhalten, wobei das Pflaster eine normalisierte Cₘₐₓ von 3,3 bis 82,5 ng/ml-(mg/h) aufweist.

18. Transdermales Pflaster nach einem der Ansprüche 1 bis 16 zur Verabreichung von Fentanyl, umfassend einen Kleber-Fentanylvorrat auf einer Trägerlage; wobei der Vorrat eine einphasige polymerische Zusammensetzung frei von ungelösten Komponenten umfasst, enthaltend einen Polyacrylat-Kleber der eine ausreichende Löslichkeit für Fentanyl aufweist, um gelöstes Fentanyl in einer Menge zu enthalten, die ausreichend ist, um eine Schmerzfreiheit in einem Menschen für mindestens drei Tage zu induzieren und aufrechtzuerhalten, wobei das Pflaster eine standardisierte Cₘₐₓ von 0,01 bis 0,2 ng/ml-cm² aufweist.

19. Transdermales Pflaster nach einem der Ansprüche 1 bis 16 zur Verabreichung von Sufentanil, umfassend einen Kleber-Sufentanilvorrat auf einer Trägerlage; wobei der Vorrat eine einphasige polymerische Zusammensetzung frei von ungelösten Komponenten umfasst, enthaltend einen Polyacrylat-Kleber der eine ausreichende Löslichkeit für Sufentanil aufweist, um gelöstes Sufentanil in einer Menge zu enthalten, die ausreichend ist, um eine Schmerzfreiheit in einem Menschen für mindestens drei Tage zu induzieren und aufrechtzuerhalten, wobei das Pflaster eine normalisierte Cₘₐₓ von 0,04 bis 10 ng/ml-(mg/h) aufweist.

20. Transdermales Pflaster nach einem der Ansprüche 1 bis 16, zur Verabreichung von Sufentanil, umfassend einen Kleber-Sufentanilvorrat auf einer Trägerlage; wobei der Vorrat eine einphasige polymerische Zusammensetzung frei von ungelösten Komponenten umfasst, enthaltend einen Polyacrylat-Kleber der eine ausreichende Löslichkeit für Sufentanil aufweist, um gelöstes Sufentanil in einer Menge zu enthalten, die ausreichend ist, um eine Schmerzfreiheit in einem Menschen für mindestens drei Tage zu induzieren und aufrechtzuerhalten, wobei das Pflaster eine standardisierte Cₘₐₓ von 0,001 bis 0,05 ng/ml-cm² aufweist.

21. Pflaster nach einem der Ansprüche 1 bis 20, das vollständig frei von einer Ratekontrollierenden Membran ist.

## Revendications

1. - Timbre transdermique monolithique pour l'administration de fentanyle, d'alfentanil, de carfentanil, de lofentanil, de remifentanil, de sufentanil ou de trefentanil à travers la peau, comprenant :
(a) une couche de support ;
(b) un réservoir disposé sur la couche de support, au moins la surface dudit réservoir en contact avec la peau étant adhésive ; ledit réservoir comprenant une composition polymère à une seule phase, exempte de composants non dissous, contenant une quantité de fentanyle, d'alfentanil, de carfentanil, de lofentanil, de remifentanil, de sufentanil ou de trefentanil suffisante pour induire et maintenir une analgésie dans un être humain pendant au moins trois jours ;
**caractérisé par le fait que** le réservoir est formé à partir d'un adhésif polyacrylate et a une épaisseur de 0,0125 mm (0,5 mil) à 0,1 mm (4 mil).

2. - Timbre selon la revendication 1, qui contient du fentanyle.

3. - Timbre selon la revendication 1, qui contient de l'alfentanil, du lofentanil, du remifentanil ou du sufentanil en tant que médicament.

4. - Timbre selon la revendication 3, dans lequel le médicament est le sufentanil.

5. - Timbre selon l'une quelconque des revendications précédentes, dans lequel ledit adhésif polyacrylate a une Tg de moins de -10°C.

6. - Timbre selon l'une quelconque des revendications 1 à 5, dans lequel ledit timbre présente une Cₘₐₓ normalisée de 3, 3 à 82,5 ng/ml- (mg/h) .

7. - Timbre selon la revendication 6, dans lequel le timbre présente un flux de médicament à l'état stable de 0,1 à 20 µg/cm²/h, de préférence de 1 à 10µg/cm²/h.

8. - Timbre selon l'une des revendications 1 à 7, dans lequel ledit timbre présente une Cₘₐₓ standardisée de 0,001 à 0,2 ng/ml-cm.

9. - Timbre selon l'une des revendications 1 à 8, dans lequel ledit réservoir comprend une quantité du médicament dissous suffisante pour induire et maintenir une analgésie pendant 3 à 7 jours.

10. - Timbre selon la revendication 9, dans lequel ledit réservoir comprend un polymère ayant une solubilité pour le médicament de 1% en poids à 25% en poids, de préférence 7% en poids à 12% en poids.

11. - Timbre selon la revendication 9 ou la revendication 10, dans lequel le réservoir comprend de 0,05 à 1,75 mg/cm², de préférence de 0,08 à 1,25 mg/cm², de façon davantage préférée 0,1 à 0,75 mg/cm², de la façon que l'on préfère le plus 0,12 à 0,5 mg/cm² du médicament.

12. - Timbre selon l'une quelconque des revendications 1 à 13, dans lequel le réservoir comprend en outre un activateur de perméation.

13. - Timbre selon l'une quelconque des revendications 1 à 12, dans lequel la couche de support comprend un polyuréthane, un poly(acétate de vinyle), un poly(chlorure de vinylidène), un polyéthylène, un poly(éthylène téréphtalate) (PET), un stratifié PET-polyoléfine ou un polymère de poly(butylène téréphtalate).

14. - Timbre selon la revendication 13, dans lequel la couche de support comprend une matière de polyéthylène basse densité (LDPE), une matière de polyéthylène moyenne densité (MDPE) ou une matière de polyéthylène haute densité (HDPE).

15. - Timbre selon la revendication 14, dans lequel la couche de support comprend une matière de polyéthylène basse densité (LDPE).

16. - Timbre selon l'une quelconque des revendications 13 à 15, dans lequel la couche de support a une épaisseur de 0,012 mm (0,5 mil) à 0,125 mm (5 mil).

17. - Timbre transdermique selon l'une quelconque des revendications 1 à 16, pour l'administration de fentanyle, comprenant un réservoir de fentanyle adhésif sur une couche de support ; ledit réservoir comprenant une composition polymère à une seule phase, exempte de composants non dissous, contenant un adhésif polyacrylate ayant une solubilité pour le fentanyle suffisante pour contenir du fentanyle dissous dans une quantité suffisante pour induire et maintenir une analgésie dans un être humain pendant au moins trois jours, ledit timbre présentant une Cₘₐₓ normalisée de 3,3 à 82,5 ng/ml- (mg/h).

18. - Timbre transdermique selon l'une quelconque des revendications 1 à 16, pour l'administration de fentanyle, comprenant un réservoir de fentanyle adhésif sur une couche de support ; ledit réservoir comprenant une composition polymère à une seule phase, exempte de composants non dissous, contenant un adhésif polyacrylate ayant une solubilité pour le fentanyle suffisante pour contenir du fentanyle dissous dans une quantité suffisante pour induire et maintenir une analgésie dans un être humain pendant au moins trois jours, ledit timbre présentant une Cₘₐₓ standardisée de 0,01 à 0,2 ng/ml-cm².

19. - Timbre transdermique selon l'une quelconque des revendications 1 à 16, pour l'administration de sufentanil, comprenant un réservoir de sufentanil adhésif sur une couche de support ; ledit réservoir comprenant une composition polymère à une seule phase, exempte de composants non dissous, contenant un adhésif polyacrylate ayant une solubilité pour le sufentanil suffisante pour contenir du sufentanil dissous dans une quantité suffisante pour induire et maintenir une analgésie dans un être humain pendant au moins trois jours, ledit timbre présentant une Cₘₐₓ normalisée de 0,04 à 10 ng/ml-(mg/h).

20. - Timbre transdermique selon l'une quelconque des revendications 1 à 16, pour l'administration de sufentanil, comprenant un réservoir de sufentanil adhésif sur une couche de support ; ledit réservoir comprenant une composition polymère à une seule phase, exempte de composants non dissous, contenant un adhésif polyacrylate ayant une solubilité pour le sufentanil suffisante pour contenir du sufentanil dissous dans une quantité suffisante pour induire et maintenir une analgésie dans un être humain pendant au moins trois jours, ledit timbre présentant une Cₘₐₓ standardisée de 0,001 à 0,05 ng/ml-cm².

21. - Timbre selon l'une quelconque des revendications 1 à 20, qui est complètement exempt d'une membrane de contrôle de la vitesse.
